# EUROPEAN PATENT APPLICATION

(11) **EP 1 852 499 A1**
(43) Date of publication of application: **07.11.2007**
(21) Application number: 06714326.3
(22) Date of filing: 22.02.2006
(51) Int. Cl.: C12N 5/02, C12N 5/06, A61K 31/202, A61K 31/557, A61L 27/00

(54) **NEUROTIZATION PROMOTING AGENT**

(30) Priority: 22.02.2005 JP 2005046203
(71) Applicant: MOCHIDA PHARMACEUTICAL CO., LTD., Shinjuku-ku Tokyo 160-8515 (JP); National University Corporation Shimane University, Matsue-shi, Shimane 690-8504 (JP)
(72) Inventor: SHIDO, Osamu, Shimane, 6930011 (JP); HASHIMOTO, Michio, Shimane, 6930022 (JP); KAWAKITA, Eisuke, a, Kiyose-shi, Tokyo, 2040022 (JP); HARADA, Tsuyoshi, -chome, Shinjuku-ku, Tokyo, 1608515 (JP)
(74) Representative: Grünecker, Kinkeldey, Stockmair & Schwanhäusser Anwaltssozietät
(86) International application number: PCT/JP2006/303186
(87) International publication number: WO 2006/090745

(57) **Abstract**

A neuronal differentiation promoting agent for neural stem cells comprising, as an active ingredient, at least one member selected from the group consisting of ω-3 unsaturated fatty acids and ω-6 unsaturated fatty acids having 18 to 22 carbon atoms, and derivatives thereof. The agent can be used for induction of differentiation of neural stem cells and is useful for treating and/or preventing a variety of neurological diseases, and in the fields of nerve transplantation and/or regenerative medicine for nerves.

## Description

### TECHNICAL FIELD

The present invention relates to a neuronal differentiation promoting agent for promoting differentiation of neural stem cells. The present invention also relates to a method of promoting differentiation of undifferentiated neural cells such as neural stem cells by using a neuronal differentiation promoting agent. The present invention further relates to a composition for regenerative medicine containing cultivated cells and a neuronal differentiation promoting agent.
The present invention also relates to a culture medium which contains the neuronal differentiation promoting agent and is adapted for undifferentiated neural cells such as neural stem cells.

### BACKGROUND ART

In recent years, regenerative medicine has attracted attention which is intended to regenerate functions of body tissues/organs with functional impairment or dysfunction by transplanting cells or stimulating endogenous, autologous cells. In terms of neural system, there is an increasing expectation for regenerative medicine for nerves as a method of treating diseases involving the damage or degeneration/decrease of cells or tissues of the central nervous system, such as cerebrovascular disorders, Parkinson's disease, Alzheimer's disease and spinal cord injury, and the importance of such medicine is also increasing.

In the 1990s, transplantation of ventral mesencephalic tissues of aborted fetuses to a Parkinson's patient has already been reported. However, this transplantation treatment requires about ten aborted fetuses per patient, and therefore has many problems to be solved, such as ethical issues, issues on the number, and cell quality issues (Non-Patent Document 1).

In this connection, neural stem cells having self-replicating ability and pluripotency are lately gaining interest. The neural stem cells can be prepared in bulk by cultivation, and can be differentiated in vitro into neurocytes or glial cells in the presence of a differentiation factor. A report shows that transplantation of neural stem cells differentiated from embryonic stem cells (ES cells) to a cynomolgus monkey with a Parkinson's disease-like symptom alleviated the symptom (Non-Patent Document 2).
If neural stem cells can be differentiated efficiently, it is possible to transplant neural stem cells to a tissue with its functions lost and differentiate the cells into a nerve tissue in vivo, or to differentiate neural stem cells into neurocytes or glial cells in vitro and transplant the resultant cells. For the purpose of treatment of Parkinson's disease and the like, it is also possible to, for example, further differentiate the cells differentiated from neural stem cells into specified neurocytes to transplant them, which provides a novel treatment means for nerve diseases.

Among factors involved in differentiation of neural stem cells, platelet derived growth factor (PDGF) is known to be a soluble factor relating to the fate determination of neural stem cells into neurons. It has been reported that insulin like growth factor (IGF-I) and brain derived neurotrophic factor (BDNF) promote the fate determination of neural stem cells into neurons and their subsequent differentiation and maturing. In addition, it has been reported that coaddition of retinoic acid (RA) and forskolin (FSK) to a culture system of the promote neural stem cells derived from a living rat hippocampus drastically promotes differentiation into neurons (Non-Patent Document 3).
Thyroid hormone (T3) has been reported as a factor relating to differentiation into oligodendrocytes, and ciliary neurotrophic factor (CNTF), or coaddition of leukemia inhibitory factor (LIF) and bone morphogenic protein-2 (BMP-2), for instance, has been reported as a factor relating to differentiation into astrocytes (Non-Patent Document 4).

However, not only the above-mentioned factors but also a plurality of other biogenic factors are considered to act on neural stem cells. The above-mentioned PDGF, IGF-I, BDNF, and the like are protein molecules, and therefore they are easily affected by many proteases present in the living body, and may cause production of autoantibody in rare cases. Considering actual treatments, it is preferable to select the optimal differentiation factor depending on cells, culture environments, environments in the body to be subjected to transplantation, patient's constitution, usage, and the like, and a novel substance with physiological activity is expected to be identified as one of the substances relating to differentiation of neural stem cells.

Unsaturated fatty acid is a substance that is known as a nutrient component required for important organs and tissues such as heart, circulatory organs, brain, and skin. In particular, EPA and DHA have effects of "decreasing neutral lipids", "suppressing platelet aggregation", and the like, and therefore they are widely used in pharmaceuticals, supplements, and so forth.

A document shows use of an unsaturated fatty acid in the field of psychiatry and neurology, and reports that administration of EPA had the effect of improving cognitive function on patients with Alzheimer's-type dementia and cerebrovascular dementia (herein also referred to as dementia/cognitive impairment) (Non-Patent Document 5).
In addition, the document titled "highly purified ethyl EPA and other EPA derivatives against psychiatrtic and neurological disorders" (Patent Document 1) describes that highly purified EPA is effective against psychiatrtic and neurological disorders such as schizophrenia, manic-depression, anxiety, dementia, and Huntington's disease.

It, however, is considered in Non-Patent Document 5 that the effect is probably realized by the action of EPA on minimal arteries or platelets to improve the cerebral blood flow. In Patent Document 1, it is described that the effectiveness of EPA results chiefly from its phospholipase A₂ inhibiting effect. In other words, neither document discloses the effect of EPA on neural stem cells.

It is reported that, when DHA was ingested by young and aged rats for a long period of time, memory and learning ability of both rats were improved. This result is considered in the report to be brought about by the antioxidant effect of DHA, and the report does not disclose the effect of DHA on neural stem cells (Non-Patent Document 6).

Patent Document 2 describes that EPA and DHA exhibit activity in enhancing the nerve growth factor (NGF) production in an established fibroblast line. However, NGF is not a factor involved in differentiation of neural stem cells, and the effects of EPA and DHA on neural stem cells are not examined in the document.

On the other hand, lipids such as linoleic acid, oleic acid, and cholesterol have been used as additives required upon culture of various cells to decrease the FBS concentration and replace FBS with them. No report, however, has been made that a fatty acid promotes neuronal differentiation.

As described above, no findings have been reported about the effects of unsaturated fatty acids including EPA and DHA on neural stem cells. In the present specification, the unsaturated fatty acids may include not only free unsaturated fatty acids but also derivatives thereof.
NON-PATENT DOCUMENT 1: Hideyuki Okano, Protein, Nucleic acid and Enzyme, Vol. 45, No. 13, 2063-2077 (2000)
NON-PATENT DOCUMENT 2: Yasushi Takagi et al., The Journal of Clinical Investigation, Vol. 15, No. 1, 102-109 (2005)
NON-PATENT DOCUMENT 3: Kin'ichi Nakashima, Protein, Nucleic acid and Enzyme, Vol. 49, No. 6, 718-726 (2004)
NON-PATENT DOCUMENT 4: Hideyuki Okano, Experimental Medicine, Vol. 20, No. 9, 1276-1302 (2002)
NON-PATENT DOCUMENT 5: Mieko Otsuka and Akira Ueki, "Analysis of dietary factors in dementia patients and study on cognitive function-improving effect of eicosapentaenoic acid (EPA)," Dementia Japan, Vol. 15, No. 1, April, 21-29 (2001)
NON-PATENT DOCUMENT 6: New technology information service (http://www.s-iri.pref.shizuoka.jp/tech/top.htm), food/biotechnology field, H14-No. 39
PATENT DOCUMENT 1: JP 2002-535355 A
PATENT DOCUMENT 2: JP 8-143454 A

### DISCLOSURE OF THE INVENTION

### PROBLEMS TO BE SOLVED BY THE INVENTION

An object of the present invention is to provide a neuronal differentiation promoting agent and a method of promoting differentiation of undifferentiated neural cells such as neural stem cells by using such an agent. A further object of the present invention is to provide a composition for regenerative medicine which is particularly useful in the field of transplantation/regenerative medicine. Another object of the present invention is to provide a culture medium having the effect of promoting differentiation of undifferentiated neural cells such as neural stem cells in cultivation of the cells.

### MEANS TO SOLVE THE PROBLEMS

The inventors of the present invention made extensive studies in order to achieve the above-mentioned objects and, as a result, discovered that ω-3 unsaturated fatty acids and ω-6 unsaturated fatty acids having 18 to 22 carbon atoms, EPA, DHA and DPA in particular, have the effect of promoting differentiation of neural stem cells, thereby having completed the present invention.

That is, a first aspect of the present invention provides a neuronal differentiation promoting agent including, as an active ingredient, at least one member selected from the group consisting of ω-3 unsaturated fatty acids and ω-6 unsaturated fatty acids having 18 to 22 carbon atoms, and derivatives thereof, preferably at least one member selected from the group consisting of eicosapentaenoic acid (EPA), docosahexaenoic acid (DHA), docosapentaenoic acid (DPA), and derivatives thereof.
A second aspect of the present invention provides a method of promoting differentiation of undifferentiated neural cells, including a step of adding the neuronal differentiation promoting agent according to the present invention.
A third aspect of the present invention provides a composition for regenerative medicine including, as a main component, the culture obtained by adding the neuronal differentiation promoting agent.
A fourth aspect of the present invention provides a culture medium for undifferentiated neural stem cells which contains the neuronal differentiation promoting agent according to the present invention.
A fifth aspect of the present invention provides a treatment method which includes using the neuronal differentiation promoting agent according to the first aspect to differentiate neural cells.
A sixth aspect of the present invention provides use of at least one member selected from the group consisting of ω-3 unsaturated fatty acids and ω-6 unsaturated fatty acids having 18 to 22 carbon atoms, and derivatives thereof for manufacturing a neuronal differentiation promoting agent.

### EFFECTS OF THE INVENTION

The neuronal differentiation promoting agent of the present invention has the effects of promoting differentiation of neural stem cells, increasing the number of differentiated cells and, in the case of differentiation into neurocytes, promoting neurite elongation. The neuronal differentiation promoting agent of the present invention retains its differentiation promoting effect for the duration of the differentiation of neural stem cells, and the present invention is useful as a novel means in regenerative medicine for inducing differentiation of neural stem cells and precursor cells safely and in a short period of time, and can be used for treatment and/or prevention of various neurological diseases and in the fields of nerve transplantation and regenerative medicine for nerves.

### BRIEF DESCRIPTION OF THE DRAWINGS

[Fig. 1] Fig. 1 is a diagram showing the photographs as alternatives to drawing which were taken on the fourth day after addition of EPA and DHA, respectively (with the photograph of the control being shown for comparison).
[Fig. 2] Fig. 2 is a diagram showing the photographs as alternatives to drawing which were taken on the seventh day after addition of EPA and DHA, respectively (with the photograph of the control being shown for comparison).
[Fig. 3] Fig. 3 is a graph showing the numbers of neurocytes per cm², with the values having been found on the seventh day after addition of EPA and DHA, respectively.
[Fig. 4] Fig. 4 is a graph showing the ratios of the number of the cells differentiated into neurocytes to the total cell number, with the values having been found on the seventh day after addition of EPA and DHA, respectively.

### BEST MODE FOR CARRYING OUT THE INVENTION

Hereinafter, the present invention will be described in detail. First, the meanings of main terms in the present invention are explained.

The term "neural stem cell" means an undifferentiated cell which is capable of proliferating and being repeatedly subcultured (self-replicating ability) and capable of giving three kinds of cells that constitute the central system: neuron, astrocyte, and oligodendrocyte (pluripotency). Examples of major specific markers include nestin and Musashi-1 (cited reference: Protein, Nucleic acid and Enzyme, Vol. 45, No. 13, 2066 (2000)).

The term "neurocyte (neuron)" means a nerve cell that has neurite and axon which are elongated, forms synapses along with an adjacent neuron, and secretes a neurotransmitter. Examples of major specific markers include β-tubulin for neurocytes, and Tuj1 for immature neurocytes.
The term "astrocyte" means a cell that is located among neurocytes and provides them with nutrients. Examples of major specific markers include GFAP (glial fibrillary acidic protein).
The term "oligodendrocyte" means a cell that wraps around an axon of a neuron to help saltatory conduction. Examples of major specific markers include GalC (galactocerebroside).

The term "precursor cell" means a cell that is incorporated into the lineage of some differentiated cell and destined to complete differentiation after a limited number of divisions. It is said that neural stem cells supply two kinds of precursor cells: neuronal precursor cells (giving neurocytes) and glial precursor cells (giving astrocytes and oligodendrocytes).
The term "undifferentiated neural cell" means a neural stem cell, a precursor cell (neuronal precursor cell, glial precursor cell), and a mixture thereof.

In the present invention, the phrase "promoting differentiation of neural stem cells" means increasing the number of the cells differentiated from neural stem cells, namely neurocytes, astrocytes or oligodendrocytes, or such cells in an immature form, or the precursors of such cells. The differentiated cells include not only the cells that can be identified morphologically but those which can be identified with the above-mentioned specific markers. In the case of differentiation into neurocytes, the promotion thereof is also determined by the elongation of neuronal processes. The above phrase may also mean increasing the cells further differentiated in a specified direction (such as dopaminergic neurons, cholinergic neurons, and motor neurons).

The term "unsaturated fatty acid" means a fatty acid having a double bond.
The ω-3 unsaturated fatty acid is a fatty acid having the first double bond at the third carbon atom as counted from the methyl group side in the structural formula. Specific examples of the ω-3 unsaturated fatty acid having 18 to 22 carbon atoms include α-linolenic acid (number of carbon atoms : degree of unsaturation) (18:3), octadecatetraenoic acid (18:4), eicosatetraenoic acid (20:4), EPA (20:5), DPA (22:5), and DHA (22:6). The ω-6 unsaturated fatty acid is a fatty acid having the first double bond at the sixth carbon atom as counted from the methyl group side in the structural formula. Specific examples of the ω-6 unsaturated fatty acid having 18 to 22 carbon atoms include linoleic acid (18:2), γ-linolenic acid (18:3), dihomo-γ-linolenic acid (20:3), and arachidonic acid (20:4).

The term "nerve regeneration" means recovery of functions of a damaged nerve. Specifically, it includes recovery of signaling via the nerve by repairing a damaged site through the following steps: differentiation of neural stem cells into neurocytes (neurons) or glial cells, production of the proteins or neurotransmitters necessary for the function, formation of axons or synapses, and the like.

The term "regenerative medicine for nerves" means a medical treatment for regeneration of functions of a nerve tissue with functional impairment or dysfunction. Examples thereof include a method of exogenously adding or transplanting neural stem cells or a nerve tissue to a damaged/lost tissue or cell region, and a method of stimulating patient's neural stem cells or precursor cells to differentiate them into neurocytes or glial cells and proliferate the resultant cells.

The neuronal differentiation promoting agent according to the first aspect of the present invention will be described.
The neuronal differentiation promoting agent of the present invention is a composition having the effect of promoting differentiation of neural stem cells and containing, as an active ingredient, an unsaturated fatty acid, preferably at least one member selected from the group consisting of ω-3 unsaturated fatty acids having 18 to 22 carbon atoms, ω-6 unsaturated fatty acids having 18 to 22 carbon atoms, and derivatives thereof, more preferably at least one member selected from the group consisting of ω-3 unsaturated fatty acids having 18 to 22 carbon atoms and derivatives thereof, still more preferably at least one member selected from the group consisting of EPA, DHA, DPA, and derivatives thereof, and especially at least one member selected from the group consisting of EPA, DHA, and derivatives thereof.
The term "at least one member" is to be understood to express that the unsaturated fatty acids as above may be used singly or as a mixture of at least two of them.
The neuronal differentiation promoting agent of the present invention has the effects of promoting differentiation of neural stem cells, increasing the number of differentiated cells and, in the case of differentiation into neurocytes, promoting neuronal process elongation.

The neuronal differentiation promoting agent of the present invention contains the unsaturated fatty acid as a free fatty acid or in the form of a derivative thereof. Derivatives of the unsaturated fatty acid include pharmacologically acceptable esters, salts, glycerides, phospholipids, amides, and any other derivatives in a pharmacologically acceptable form. Derivatives in ester or salt form are particularly suitable for use.

Examples of the pharmacologically acceptable unsaturated fatty acid ester used in the present invention include, but are not limited to, such compounds as methyl ester, ethyl ester, propyl ester and butyl ester of the unsaturated fatty acid of interest, and ethyl ester is preferable.
Examples of the pharmacologically acceptable salt which may be used include: a pharmacologically acceptable salt with an inorganic base, such as sodium salt and potassium salt; that with an organic base, such as benzylamine salt and diethylamine salt; and that with a basic amine acid, for instance, basic amino acid, such as arginine salt and lysine salt.
A fatty acid glyceride may also be employed which is an ester of the unsaturated fatty acid of interest and glycerine. Examples of the fatty acid glyceride include triglyceride, diglyceride, monoglyceride, a mixed glyceride substituted with a fatty acid other than the unsaturated fatty acid of interest, and a mixture thereof.
Further, the unsaturated fatty acid of interest may be used in the form of glycerophospholipid, amide, or the like.

The unsaturated fatty acid serving as an active ingredient in the present invention can be prepared by a known method such as extraction or purification from a natural product, or chemical synthesis. The unsaturated fatty acid may be a commercially available product, or may be obtained by the purification from fish oil, or from EPA-producing bacteria, DHA-producing bacteria or a culture solution containing such bacteria, using a known method such as continuous distillation, urea addition, liquid chromatography, supercritical fluid chromatography, or a combination thereof.

The purity of the unsaturated fatty acid serving as an active ingredient in the present invention is not particularly limited, and the unsaturated fatty acid may be a product of lower purity obtained in the process of purification from fish oil. In that case, the unsaturated fatty acid preferably comprises at least 20% by mass, more preferably at least 50% by mass, still more preferably at least 85% by mass, and even more preferably at least 95% by mass of the total fatty acid mass. It, however, is particularly preferable that the unsaturated fatty acid is used alone, that is to say, is substantially free from other fatty acid components. Preferred as an active ingredient in the present invention are ω-3 polyunsaturated fatty acids, especially EPA and/or DHA, and more preferred is eicosapentaenoic acid ethyl ester (EPA-E) and/or docosahexaenoic acid ethyl ester (DHA-E) as a derivative.
As for EPA, an existing, commercially available product may be used, such as eicosapentaenoic acid (free) with a purity of about 99% (manufactured by Nacalai Tesque, Inc. or sigma Corp.), eicosapentaenoic acid sodium salt with a purity of about 90% (manufactured by,sigma Corp.), and eicosapentaenoic acid ethyl ester with a purity of about 90% (manufactured by Tokyo Chemical Industry Co., Ltd.). Note that high-purity EPA-E preparations with their respective trade names Epadel and Epadel S (both manufactured by Mochida Pharmaceutical Co., Ltd.) are commercially available in Japan as therapeutic agents for arteriosclerosis obliterans and hyperlipidemia.
As for DHA, docosahexaenoic acid (free) with a purity of 98% or more (manufactured by Cayman Chemical Company), docosahexaenoic acid ethyl ester with a purity of about 95% (manufactured by Tokyo Chemical Industry Co., Ltd.), and the like are usable.
A soft capsule containing about 46% by weight of EPA-E and about 38% by weight of DHA-E (Omacor (Ross Products)) has already been commercialized in the U.S.A. and other countries as a therapeutic agent for hypertriglyceridemia. Such a preparation may also be employed.

The neuronal differentiation promoting agent of the preset invention may further contain a factor having a certain effect on cells, and other substances, if necessary.

The factor having a certain effect on cells is a factor inducing differentiation of neural stem cells, a factor inhibiting self-reproduction of neural stem cells, a growth factor for differentiated neurocytes, or the like. Examples thereof include, but are not limited to, platelet growth factor (PDGF), insulin-like growth factor (IGF-I), brain derived neurotrophic factor (BDNF), coaddition of retinoic acid (RA) and forskolin (FSK), transforming growth factor β (TGFβ), NGF, ciliary neurotrophic factor (CNTF), NT-3, and NT-4/5.

The unsaturated fatty acid serving as an active ingredient in the present invention is fat-soluble and therefore is difficult to blend in a culture solution. The neuronal differentiation promoting agent of the present invention may contain a compound having an ability to make a fatty acid blended in a culture medium. Use of the compound facilitates the action of the neuronal differentiation promoting agent on neural stem cells and allows the agent to exert its effect easily. Examples of the compound having the above ability include albumin, phospholipids such as phosphatidylcholine, liposomes, lipoproteins, and surfactants (such as Tween 80), whereupon fatty acid-free albumin is preferable.

Since the unsaturated fatty acid serving as an active ingredient in the present invention is highly unsaturated, the differentiation promoting agent of the present invention can contain an antioxidant in an amount effective at suppressing oxidation of the unsaturated fatty acid. Examples of the antioxidant-which may be used include butylated hydroxytoluene (BHT), butylated hydroxyanisole (BHA), propyl gallate, gallic acid, pharmaceutically acceptable quinone, and α-tocopherol.

Apart from the above-mentioned substances, the neuronal differentiation promoting agent of the present invention may contain a physiologically acceptable carrier, medium, and so forth, as well as a variety of additives in an appropriate combination. For example, the agent may contain vehicle, binder, colorant, sterilized water, vegetable oil, inorganic organic solvent, harmless solubilizer (such as glycerin or propylene glycol), emulsifier, suspending agent (such as Tween 80 or gum arabic solution), isotonicity, pH adjuster, stabilizer or soothing agent, if necessary.

The neuronal differentiation promoting agent of the present invention is supplied in various forms depending on its usage. Exemplary forms include those for the addition to a culture solution, those for the administration upon transplantation, and pharmaceutically acceptable dosage forms such as for injection or oral administration. Examples of the dosage form of a preparation include, but are not limited to, liquid, emulsion, suspension, nonaqueous solution, tablet, capsule, microcapsule, liquid preparation for oral use, and injection (emulsion-type, suspension-type, nonaqueous). In order to promote differentiation of neural stem cells more effectively, the agent may be a sustained-release preparation obtained by a method known to those skilled in the art.

The neuronal differentiation promoting agent of the present invention is used in various fields for the purpose of promoting differentiation of undifferentiated neural cells such as neural stem cells. The agent, however, is preferably used for regenerative medicine.
Regenerative medicine for nerves has been described in the explanation of terms, and the neuronal differentiation promoting agent of the present invention can be used singly or in combination with the factor having a certain effect on cells as described before, or even in combination with other therapeutic agents or treatment methods, and can play some role in the transplantation/regenerative medicine for nervous systems.
The neuronal differentiation promoting agent of the present invention is used in, for example, preparation of neural cells in a suitable differentiation state to be used for transplantation in regenerative medicine for nerves. In that case, the agent may be used in one out of a series of processes for obtaining specified cells suitable for transplantation. It is also possible to administer the agent directly to the living body in a treatment aimed at differentiation of undifferentiated neural cells.
Detailed usage of the present invention will be described later.

The differentiation promoting agent of the present invention can be used for treating various cerebrovascular disorders or neural diseases, such as nervous system diseases caused by loss/damage in nerve tissues or cells, nerve degenerative diseases in cerebrovascular or senile dementia/cognitive impairment, Alzheimer's disease, and the like, amyotrophic lateral sclerosis (Lou Gehrig's disease), and Parkinson's disease, or treating traumatic spinal cord injury and the like. The neuronal differentiation promoting agent of the present invention can be applied to the above-mentioned diseases not only of human but also of other mammals (such as mouse, rat, rabbit, dog, cat, cow, and pig).

The neuronal differentiation promoting agent of the present invention can be applied to the neural stem cells of all animals including human and monkey, and the origin of the cells is not limited. In other words, neural stem cells may be obtained by: cultivating the cells separated from a fetus or living brain of any animal; inducing differentiation from ES cells; or carrying out transdifferentiation from the stem cells other than neural stem cells, and the agent can be used for any neural stem cells prepared by a known method of preparing neural stem cells (see Biomedicine & Therapeutics, vol. 38, No. 10, 2004, for example). The neuronal differentiation promoting agent of the present invention can be used at least for promoting differentiation from neural stem cells into neurocytes.
Examples of the method of preparing neural stem cells include the neurosphere method (Journal of Neuroscience, Vol. 12, 4565-4574). The neurosphere method is a method developed by Reynolds et al. in 1992 in which cells are collected from the central nervous system and cultivated in a serum-free medium in the presence of a growth factor such as epidermal growth factor (EGF) or basic fibroblast growth factor (bFGF). Neural stem cells among the collected cells proliferate in response to the growth factor to form cell clusters (neurospheres), which are separated and cultivated.

Hereinafter, the method of promoting differentiation of undifferentiated neural cells according to the second aspect of the present invention will be described.

The method of the present invention includes the step of adding the neuronal differentiation promoting agent of the present invention and is used for promoting differentiation of undifferentiated neural cells. The undifferentiated neural cells have been described in the explanation of terms, and it is particularly preferable that they are neural stem cells. The undifferentiated neural cells are prepared by a known method. In the step of adding the neuronal differentiation promoting agent of the present invention, the neuronal differentiation promoting agent is added to a culture solution containing the undifferentiated neural cells in an appropriate amount, or the cells are added to a culture solution containing the neuronal differentiation promoting agent. The neuronal differentiation promoting agent is added as such, or after modification with an appropriate diluent or the like. In this process, conditions for differentiation of the cells are adjusted by removing proliferative factors for the undifferentiated cells, adding a factor that inhibits the proliferative factors, and the like. By adding the differentiation promoting agent after adjusting the differentiation conditions, differentiation is started. After the start of differentiation induction, the cells are cultivated in the medium containing the neuronal differentiation promoting agent (medium for differentiation induction) for an appropriate period of time.

The culture density of neural stem cells and precursor cells is not particularly limited as long as these cells can proliferate/differentiate, and the culture density is 1 × 10² to 1 × 10⁹ cells/cm², preferably 1 × 10³ to 1 × 10⁷ cells/cm², and more preferably 1 × 10⁹ to 1 × 10⁶ cells/cm².

Cultivation is performed in accordance with a known method such as using a culture plate coated with an adhesion factor. The medium for differentiation induction is not particularly limited as long as it is a medium where undifferentiated neural cells can differentiate. A known synthetic medium for cell cultivation (such as Dulbecco's Modified Eagle's Medium (DMEM)) may be used and, besides the neuronal differentiation promoting agent, supplement, hormone such as insulin, carrier such as transferrin, antioxidant such as mercaptoethanol, trace element, and so forth may appropriately be added, if necessary. In addition, another factor that has a certain effect on cells may be added at the time of, or before or after addition of the neuronal differentiation promoting agent as long as differentiation of the cells is not inhibited. The medium is preferably exchanged about once every 2 days. The temperature and CO₂ content may be set at such values as generally employed in cell culture, which are 37°C and 5% CO₂, respectively.

As described above, the unsaturated fatty acid used is fat-soluble and therefore is difficult to blend in a culture solution. Accordingly, in the case where the neuronal differentiation promoting agent of the present invention does not contain a compound having an ability to easily blend a fatty acid in a culture medium, it is preferable to add the neuronal differentiation promoting agent to a medium containing fatty acid-free albumin, phospholipid such as phosphatidylcholine, liposome, lipoprotein, surfactant (such as Tween 80) or the like, or to add a mixture of the neuronal differentiation promoting agent and any of the above-mentioned compounds to the medium. The amount of the compound added is not particularly limited as long as the compound does not lead to toxicity to cells, and in the case of albumin, its final concentration is preferably 0.05 to 0.5%, and more preferably 0.1 to 0.3%.

The period of differentiation induction is not particularly limited, and the cells are cultivated for an appropriate number of days depending on circumstances of differentiation of the cells. In the case of neurocytes, neurite elongation occurs with an increase in the number of days for cultivation, and therefore differentiation induction is stopped when the cells differentiate to a level suitable for a desired application. For example, in the neurocytes, the neuronal process elongation occurs when cultivation is continued for 2 to 7 days. The number of days for cultivation may be determined by detecting a differentiation marker for neural cells such as Tuj1 or GFAP, and evaluating circumstances of differentiation based on the marker serving as an index.

In the case of adding the neuronal differentiation promoting agent of the present invention to a medium, the concentration of the unsaturated fatty acid as an active ingredient of the neuronal differentiation promoting agent is 0.1 to 100 µM, preferably 0.1 to 20 µM, and a concentration of 1 to 15 µM is particularly preferable.

The method of the present invention is preferably performed in vitro, but it may be performed in vivo. For example, the neuronal differentiation promoting agent is administered to the region in the brain or spinal cord of a patient with a cerebrovascular disorder, Alzheimer's, or Parkinson's disease where neural tissues or cells are lost/damaged at the time of, or before or after transplantation of a graft containing undifferentiated cells such as neural stem cells, neuronal precursor cells and glial precursor cells. The administration mode of the neuronal differentiation promoting agent is not particularly limited, and the agent may be injected or continuously injected (using an osmotic pump as required), or may be allowed to permeate into an appropriate instrument to transplant the resultant instrument together with the graft.

The above administration or transplantation is preferably carried out in the region where neural tissues or cells are lost/damaged and/or the vicinity of the region. It, however, has been reported that neural stem cells extend for themselves to a damaged region in the brain, so that the position of administration or transplantation is not necessarily limited to the vicinity of the damaged region. The neuronal differentiation promoting agent of the present invention may be directly administered to endogenous undifferentiated neural cells or to the vicinity of the cells for the purpose of proliferation and differentiation of the cells.

As described above, the differentiation promoting agent of the present invention may be administered by a selected appropriate administration method in a treatment aimed at differentiation of undifferentiated neural cells. Preferable is topical use where the unsaturated fatty acid concentration around undifferentiated cells is more easily controlled, more preferable is use with transplantation of cells where conditions (such as cell composition) are easily adjusted so that they may be suitable for nerve regeneration, and particularly preferable is topical use with transplantation of cells.

In the case where the neuronal differentiation promoting agent of the present invention is directly administered to the brain or spinal cord, it is desirable that the unsaturated fatty acid concentration in the region of undifferentiated neural cells is kept at 0.1 to 100 µM, preferably 0.1 to 20 µM, and particularly 1 to 15 µM, until the cells have differentiated. In the case of oral administration, the agent is administered at a dose of 0.1 to 9 g/day, preferably 0.5 to 6 g/day, and more preferably 1 to 3 g/day in terms of unsaturated fatty acid in three divided portions. If necessary, the total dose may be administered at a time or in several divided portions. In the case of intravenous or intraarterial administration, the agent is administered at a dose of 1 to 200 mg, preferably 5 to 100 mg, and more preferably 10 to 50 mg in terms of unsaturated fatty acid at a time or in several divided portions, and may be administered continuously over several hours to several days using a drip or the like., if necessary.
The number of administration and the dosage may appropriately be increased or decreased depending on the severity of the symptom, body weight, age, and the like.

Next, the composition for regenerative medicine according to the third aspect of the present invention will be described.
The composition for regenerative medicine of the present invention is a composition containing, as a main component, the culture obtained by adding the neuronal differentiation promoting agent of the present invention to neural stem cells. In this regard, the culture refers to: the neural stem cells and the neuronal differentiation promoting agent of the present invention; the cells obtained by adding the neuronal differentiation promoting agent to the neural stem cells and then cultivating the neural stem cells for a given period of time, and the neuronal differentiation promoting agent; or a culture mixture obtained by adding the neuronal differentiation promoting agent to the neural stem cells and then cultivating the neural stem cells for a given period of time. The composition of the present invention is a cell composition that contains at least one out of the above three and contains cells for transplantation to be used for regenerative medicine. The composition for regenerative medicine of the present invention can be made more suitable for transplantation by adding any ingredient described below to the composition containing the above-mentioned culture.

The cells in the composition for regenerative medicine of the present invention are not particularly limited either in animal species or direct origin from which they are derived, but the cells are preferably derived from the same species as the animal to be subjected to transplantation.
As described above, the neural stem cells can be prepared by a known method.
The method of adding the neuronal differentiation promoting agent of the present invention to neural stem cells to cultivate the cells is as mentioned above. The period of time for cultivation is not particularly limited. From a resultant cultivation product, one or several kinds of target cells are selected based on morphological alterations or markers expressed on cell surfaces as indexes. Specific examples of the target cell include: one member selected from the group consisting of neuronal precursor cells, glial precursor cells, neurocytes, astrocytes, oligodendrocytes, and cells that further differentiate in a specified direction (such as dopaminergic neurons, cholinergic neurons, and motor neurons); and a mixture of any two or more members. Preferable cells to be selected are neuronal precursor cells, glial precursor cells, and neurocytes.
The culture mixture is a product obtained by adding the neuronal differentiation promoting agent of the present invention to neural stem cells to cultivate the cells for a given period of time, and includes cells and a culture solution. The cells in the culture mixture are any one kind of the cells listed above, or a mixture thereof, and may include initial neural stem cells. The culture solution includes the neuronal differentiation promoting agent of the present invention.

The composition for regenerative medicine of the present invention can be prepared by: adding the neuronal differentiation promoting agent of the present invention to neural stem cells, or to the cells obtained by adding the neuronal differentiation promoting agent of the present invention to neural stem cells to cultivate the cells for a given period of time; suspending the cells in an appropriate solvent and adding the neuronal differentiation promoting agent of the present invention to the solvent; allowing the cells to adhere to a culture plate or the like including a solvent that contains the neuronal differentiation promoting agent of the present invention; allowing the cells to adhere to an instrument in which the neuronal differentiation promoting agent permeates; or the like. Further, the composition may be a culture mixture itself obtained by cultivating neural stem cells in a medium containing the neuronal differentiation promoting agent. The neuronal differentiation promoting agent of the present invention is added so that the unsaturated fatty acid concentration in a cell region may be 0.1 to 100 µM, preferably 0.1 to 20 µM, and particularly 1 to 15 µM. In addition, it is preferable to confirm the absence of cells other than target cells (such as cancer cells) among the above-mentioned cells or in a culture mixture by utilizing the expression of a tumor marker or morphological characteristics as an index and, if necessary, remove such cells before use.

The solvent is preferably one that is physiologically suitable to both neural cells and body environment. Examples thereof include physiological saline and phosphate buffered saline (PBS). The solvent may be basic medium (BM) including buffers and such nutrients as amino acids, sugars and vitamins, Dulbecco's Modified Eagle's Medium (DMEM), neurobasal medium, Hanks' Balanced Salt Solution (HBSS), or the like, and if necessary, it may contain a factor having an effect on cells (such as BDNF or NGF), vitamins, supplements for cell culture (such as B27), hormones (such as insulin and progesterone), carriers (such as transferrin, serum albumin, and lipoprotein), antioxidants (such as mercaptoethanol and Se), antibiotics (such as penicillin/streptomycin), other cells that are expected to provide an effect of further promoting nerve regeneration when used together with the above-mentioned cells (such as neural cells prepared separately), a culture mixture thereof, and the like.

The composition for regenerative medicine of the present invention is provided in any form. For example, the composition may be provided in the form of a plate with cells attached, a suspension of cells in a solvent, a culture mixture, an instrument or gel with cells attached (preferably biodegradable instrument or gel), or in a form suitable for sustained release including a capsule and a self-contained pump, or any other form.
The composition may also be provided as one containing any of the later-mentioned vehicles, diluents, fillers, antiaggregating agents, lubricants, wetting agents, emulsifiers, antiseptics, carriers, and the like.

The composition for regenerative medicine of the present invention can be transplanted and injected in the region of the brain, spinal cord, or the like where a nerve is damaged or lost, or in the vicinity thereof, in accordance with a known method, and may be administered via various routes including surgical stereotactic introduction, intralesional introduction using a catheter, or the like. Specific examples of a method of transplanting and injecting the composition into brain or spinal cord include the method of Douglas Kondziolka (Douglas Kondziolka et al., Neurology 55: 556-569, 2000). Specifically, the patient's skull is cut to provide an opening with a diameter of about 1 cm, and the composition for nerve regeneration is injected into about three regions. In this procedure, the injection is performed using a syringe with a long needle and a stereotactic frame.

The unit dose is not particularly limited and can be determined considering a disease to be treated, symptom, age and sex of a patient, administration route, and the like. Preferably, the unit dose is within a range of about 1 × 10² to 1 × 10⁹ cells in terms of the number of the cells in the composition for regenerative medicine of the present invention.

In order to improve adhesion, survival and functions of cells, the composition for regenerative medicine of the present invention can be used by mixing differentiation-induced cells or a culture mixture with a pharmaceutically acceptable vehicle or carrier or diluting the cells or culture mixture with a pharmaceutically acceptable diluent in accordance with a conventional method.
Examples of the carrier, vehicle, and diluent include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, polylactic acid, polyglycolic acid, polycaprolactone acid, water, methyl hydroxybenzoate, propyl hydroxybenzoate, talc, magnesium stearate mineral oil, fibronectin, laminin, collagen, chitosan, elastin, glycosaminoglycan, and proteoglycan.
In addition, the composition may be used together with a filler, antiaggregating agent, lubricant, wetting agent, emulsifier, antiseptic, or the like.

The composition for regenerative medicine may be used singly or in combination with a factor having an effect on cells, and in the case of combined use, the composition and factor may be administered separately with a time lag or simultaneously. The composition for regenerative medicine of the present invention may be applied to the above-mentioned diseases of not only human but also other mammals (such as mouse, rat, rabbit, dog, cat, caw, and pig).

Next, the culture medium according to the fourth aspect of the present invention will be described.
The medium of the present invention is a medium that contains a neuronal differentiation promoting agent of the present invention, has a composition that enables growth of neural cells, and has the effects of promoting differentiation of neural stem cells, increasing the number of differentiated cells and, in the case of differentiation into neurocytes, promoting neurite elongation. The medium of the present invention is used for promoting differentiation of not only neural stem cells but also undifferentiated neural cells. The medium of the present invention can be prepared by adding an arbitrary amount of a neuronal differentiation promoting agent of the present invention to a basal medium, i.e., any medium physiologically suitable to neural cells. Examples of the basal medium include basic mediums (BM) including buffers and nutrients such as amino acids, sugars and vitamins, known synthetic mediums for cell culture such as Dulbecco's Modified Eagle's Medium (DMEM), Eagle's Minimum Essential Medium (EMEM), Iscove's Modified Dulbecco Medium (IMDM), and neurobasal mediums. In the case where the culture medium of the present invention is used for regenerative medicine, the medium is preferably one that is aseptic, virus-free, and physiologically suitable to body environment.
If necessary, the medium may further contain a factor having an effect on cells as described before, vitamin, supplement for cell culture (such as B27), hormone (such as insulin and progesterone), carrier (such as transferrin, serum albumin, and lipoprotein), antioxidant (such as mercaptoethanol and Se), antibiotic (such as penicillin/streptomycin) or the like.
In the case where the neuronal differentiation promoting agent does not contain a compound having an ability to easily blend a fatty acid in a culture medium, the medium of the present invention preferably contains fatty acid-free albumin, phospholipid such as phosphatidylcholine, liposome, lipoprotein, surfactant (such as Tween 80), or the like.

The medium of the present invention may be used for cell culture without dilution or may be used after diluted with an appropriate buffer. The concentration of the unsaturated fatty acid as an active ingredient of the neuronal differentiation promoting agent in the medium is not particularly limited as long as differentiation of neural stem cells can be promoted. It is desirable that the concentration during cultivation be 0.1 to 100 µM, preferably 0.1 to 20 µM, and more preferably 1 to 15 µM.

The treatment method including differentiating neural cells using the neuronal differentiation promoting agent of the present invention according to the fifth aspect of the present invention will be described. The treatment method of the present invention includes the method of promoting differentiation of undifferentiated neural cells according to the second aspect of the present invention at least as one step of the treatment. The usage form, administration route, and administration method of the neuronal differentiation promoting agent of the present invention are as described before with respect to the second aspect of the present invention. The method is applied to a patient with lost/damaged neural tissues or cells for the purpose of nerve regeneration. Diseases particularly suitable to the treatment are described above. A plurality of treatment methods according to the present invention may be combined, or the method of the present invention may be combined with another treatment method.

Hereinafter, the present invention will be described specifically by way of Experimental Examples and Example. The present invention is not limited thereto.

### Experimental Example 1: Differentiation promoting effect of EPA and DHA in rat neural stem cells

### (1) Preparation of neural stem cells

The telencephalons of E15 embryos (embryonic day 15) were separated from different four parent rats and cultivated by the neurosphere method (cited reference: Journal of Neuroscience, Vol 12, 4565-4574) at 37°C in 5% CO₂ for 1 week. N2 medium containing 20 ng/mL bFGF (basic fibroblast growth factor) (R & D SYSTEM 233-FB) and heparin (2 µg/mL) was used as a culture solution. The N2 medium was prepared by adding various reagents to 4 × DMEM/F12 (1:1) to achieve a final composition of N-2 supplement (100 ×) (Invitrogen), 0.6% glucose, 0.11% sodium bicarbonate solution, 2 mM L-Glutamine, 5 mM HEPES buffer solution (Invitrogen), and 25 µg/mL insulin, followed by adjustment with sterilized water. The resultant neurospheres were collected and pipetted to produce a single cell suspension, and the cells were used as neural stem cells in the following experiment.

### (2) Differentiation induction

A 15 µg/mL poly-L-ornithine solution (Sigma.) was added to a 24-well plastic plate (culture area: 2 cm²), and the plate was allowed to stand at 37°C overnight and washed with sterilized water three times to coat the plate. The plate was washed, and then 450 µL of N2 medium and 50 µL of N2 medium containing 100 µM EPA or DHA and 1% fatty acid-free bovine serum albumin (hereinafter, abbreviated as FA-free BSA) (CALBIOCHEM) were added to respective wells, followed by incubation at 37°C. N2 medium containing 0.1% FA-free BSA only was added to a control group. The procedures were repeated again.
On the other hand, the neural stem cells prepared in the procedure (1) were suspended in N2 medium and added to the above-mentioned respective wells to contain 2 × 10⁵ cells. The cells were cultivated at 37°C in the presence of 5% CO₂ to induce differentiation of neural stem cells, and measurement was performed on day 4 and day 7. Note that the medium was exchanged on day 2 and day 4.

### (3) Immunohistochemical staining

On day 4 and day 7 after initiation of differentiation induction, cells were fixed with 4% paraformaldehyde and washed with TBS for 10 minutes three times, followed by blocking with 3% normal goat serum. Anti-mouse Tuj1 (R & D SYSTEM) was added as a primary antibody and allowed to stand at 4°C overnight. The primary antibody was removed by washing with TBS twice for 10 minutes, and FITC conjugated goat anti-mouse IgG (Southern Bio.) was added as a secondary antibody, followed by a treatment for one hour at room temperature. The secondary antibody was removed by washing with TBS twice for 10 minutes, the cells were treated with TBS containing 2 µg/mL propidium iodide (PI) (Dojindo Laboratories) and 0.25% Triton X-100 for 30 minutes at room temperature, and washed with TBS twice for 10 minutes, followed by nuclear staining.

### (4) Data analysis

Seven random fields per well were sampled and observed using confocal scanning laser microscopy (Flow view FV300, Olympus Corporation), and images were analyzed with NIH image software or Adobe Photoshop. The number of PI-positive cells and number of Tuj1-positive cells per field were counted, and averages of the numbers in the seven fields were used as data of the respective wells. The number of the PI-positive cells represents the number of total cells, while the number of the Tuj1-positive cells represents the number of cells differentiated into neuron.
Statistical analyses were performed by One-way ANOVA and Bonferroni post-hoc analysis.

### (5) Results

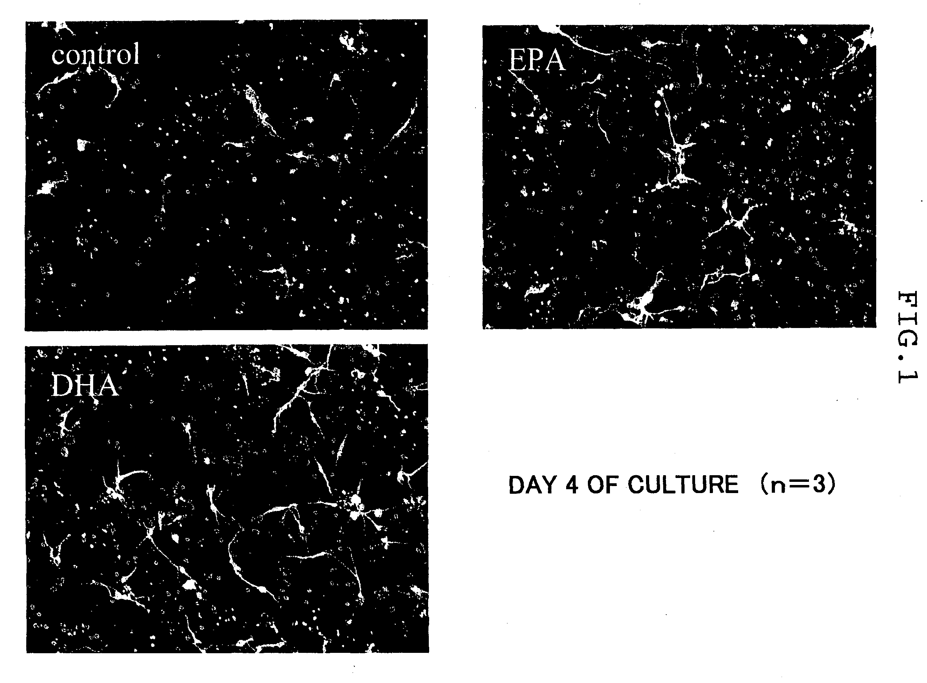

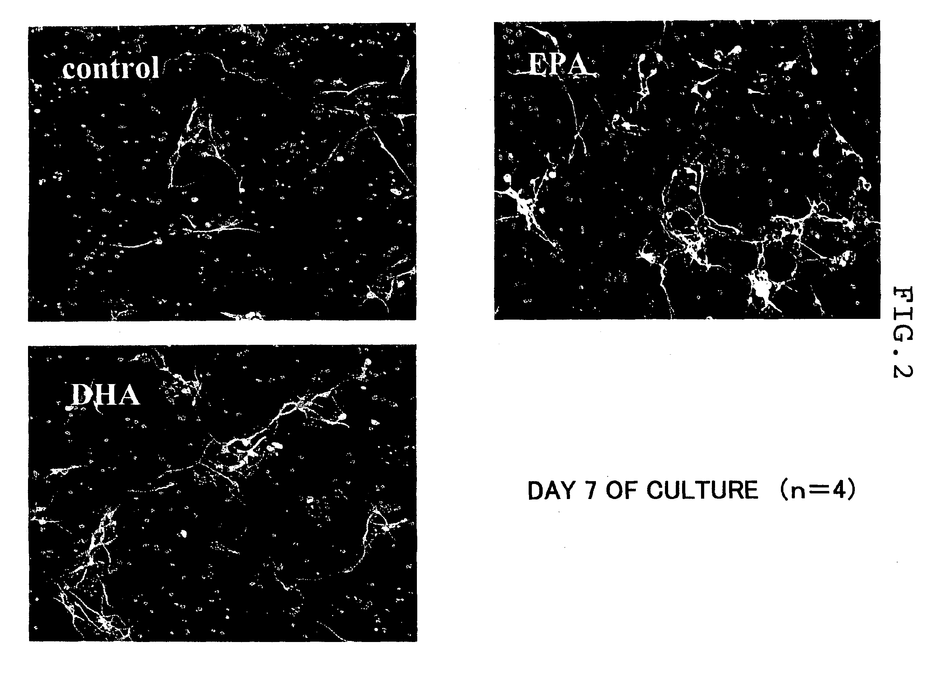

Photographs of cells of EPA-treated group, DHA-treated group, and control group on day 4 and day 7 are shown in Figs. 1 and 2. As is clear from Figs. 1 and 2, in the case of the control group, the number of cells differentiated into neuron was small, while in the cases of the EPA-added group and DHA-added group, an increase in the number of differentiated cells and neurite elongation were observed. Therefore, EPA and DHA were found to have an effect of promoting differentiation of neural stem cells.

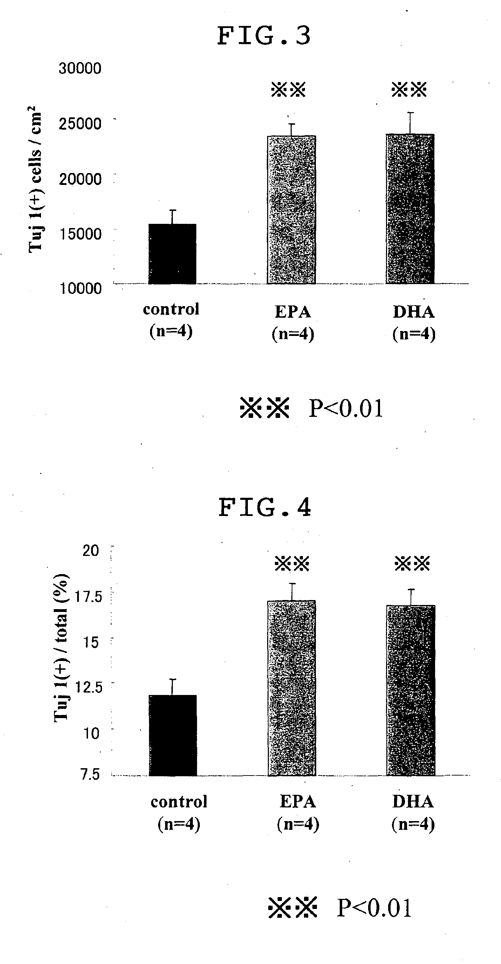

Fig. 3 shows the numbers of neuron per 1 cm² on day 7 after addition of EPA or DHA. In both the cases of EPA and DHA, the numbers of cells differentiated into neurocytes were found to significantly increase compared to the control group.
Fig. 4 shows the ratios of the number of cells differentiated into neurocytes to the number of total cells on day 7 after addition of EPA or DHA. In both the cases of EPA and DHA, the ratios of cells differentiated into neurocytes significantly increase compared to the control group.

The results confirmed that EPA and DHA have an effect of promoting differentiation of neural stem cells and an effect of promoting neurite elongation. The results are considered as typical results of an effect of an ω-3 unsaturated fatty acid having 18 to 22 carbon atoms and an ω-6 unsaturated fatty acid having 18 to 22 carbon atoms on neural stem cells.
Some of the factors having an effect on cells may act on only cells in a limited period and negatively act on the cells in a subsequent period. However, in the above-mentioned experiment, neural differentiation was observed not only on day 4 but also on day 7, and therefore the effect of promoting differentiation of EPA and DHA was found to continue during these periods. The results revealed that these unsaturated fatty acids did not inhibit differentiation of cells in these periods and had the effect of promoting differentiation not only on neural stem cells but also precursor cells. Therefore, it was suggested that, in the case of use for regenerative medicine and the like, differentiation into neurocytes, glial cells, or the like can be induced without removing these added unsaturated fatty acids.

### Referential Example 1: Effect of DHA on proliferative activity of neural stem cells

In the same way as Experimental Example 1, neural stem cells were divided into DHA group and control (DHA free) group, and 10 µM 5-bromo-2'-droxyuridine (BrdU; Sigma) was added under culture conditions for differentiation induction, followed by cultivation for 24 hours. Thereafter, the cells were treated with 2N HCl at 37°C for 10 minutes and treated with 0.1 M borate buffer (Na2B4O7, pH 8.5) for 5 minutes. The cell were treated with anti-mouse BrdU antibody (1:1000 Chemicon) and then treated with FITC-labeled secondary antibody.
7 random fields per well were sampled, and the numbers of BrdU-positive cells were counted to calculate a ratio of the number of BrdU-positive cells to the number of total cells. The number of total cells was counted by PI staining. Statistical analysis was evaluated by student's t-test.
As a result, a ratio of the BrdU-positive cells of the DHA group was found to be lower than that of the control group (control group: 23.6 ± 2.2%, n=6; DHA group: 16.3 ± 1.0%, n=6; P=0.02). There is no difference between the two groups in the numbers of total cells, and therefore DHA was considered to have an effect of suppressing the proliferation of neural stem cells in the early stage of differentiation of neural stem cells, which was consistent with the results of Experimental Example 1 showing that EPA and DHA had an effect of inducing differentiation of neural stem cells.

### Referential Example 2: Effect of DHA on apoptosis of neural stem cells and cells differentiated from neural stem cells

In the same way as Experimental Example 1, neural stem cells were divided into DHA group and control (DHA free) group and cultivated under culture conditions for differentiation induction for 7 days, and pycnotic cells were evaluated by PI staining. The pycnotic cells were found to have characteristic shapes such as condensed nuclei and constricted cells and exhibit an image of weakly PI-stained cells.
Apoptosis in the pycnotic cells were confirmed by TUNEL staining. The TUNEL staining was performed using Red In Situ Apoptosis Detection kit (Chemicon). Cells of the DHA group and the control group were cultivated for 7 days under the above-mentioned differentiation conditions and fixed with 4% paraformaldehyde, followed by incubation together with digoxigenin-dUTP in the presence of TdT at 37°C for 1 hour. TUNEL-labeled cells were detected with Rhodamine conjugated anti-digoxigenin antibody and observed using confocal scanning laser microscopy.
The TUNEL staining was performed to specifically label the 3'-OH end in a DNA helix structure disrupted by apoptosis in the cells, and the pycnotic cells were found to be TUNEL staining positive, while cells having complete shapes were confirmed to be TUNEL staining negative. Apoptosis of the cells that exhibited pycnotic-like feature stained by PI was confirmed by the TUNEL staining.
The results of the observation revealed that the ratio of the pycnotic cells to the number of total cells of the DHA group was lower than that of the control group (control group: 60.0 ± 3.2%, n=6; DHA group: 54.3 ± 3.7%, n=6; P=0.01). There were no differences in the numbers of total cells of the both groups.

### Referential Example 3: Effect of DHA on neurogenesis in adult rat hippocampal dentate gyrus region

18-week-old Wister male rats fed with a fish oil-free diet for three generations were used. To DHA group, 4,7,10,13,16,19-docosahexaenoate suspended in a 5% gum arabic solution was gavaged at 300 mg/kg/day every day for 7 weeks. To control group, in the same way as above, only a 5% gum arabic solution was gavaged in the same dose. From 2 weeks after initiation of oral administration, BrdU dissolved in PBS (-) was injected to rats intraperitoneally once a day for 5 days (50 mg/kg).
4 weeks after completion of BrdU administration, PBS (-) was perfused from the hearts of the rats, followed by fixation with 4% paraformaldehyde. The brains were isolated and fixed with paraformaldehyde for further 24 hours, followed by exchange for 25% sucrose. Coronal sections (40 µm) were cut by a microtome, and nuclei labeled with BrdU were detected by the following procedures. Slices of the brains were treated by Flating method with 50% formaldehyde-2 × SSC (0.3 M NaCl and 0.03 M sodium citrate) at 65°C for 2 hours, and incubated with 2N HCl at 37°C for 30 minutes. Thereafter, they were treated with 0.1 M boric acid (pH 8.5) and incubated with 3% goat serum in TBS containing anti-rat BrdU antibody (Oxford Biotech.) and 0.3% Triton-X100 for 24 hours. After washing with TBS, Cy5-labeled secondary antibody was added, followed by incubation at 25°C for 1 hour. They were treated with anti-mouse NeuN antibody (Chemicon) and then treated with FITC-labeled secondary antibody. In the same way as above, they were observed using confocal scanning laser microscopy.
Six sections were selected at intervals of 240 µm from the rostro caudal region of a granular cell layer in the whole dentate gyrus, and BrdU-NeuN double positive cells in the granular cell layer and subgranular zone (inside zone along the granular cell layer) were counted. Regions around the six sections were stained with 2 µg/mL PI to confirm that there were no significant differences in the numbers of total cells in the sections. The referential volume of the granular cell layer was calculated from the areas of the sampled sections.
The number of the BrdU-NeuN double positive cells in the whole granular cell layer of the dentate gyrus of the DHA group was found to be 1.6-fold compared to the control group (average number of the BrdU-NeuN double positive cells in the respective sections, n=3, control group: 538 ± 60.2 cells; DHA group: 878 ± 72.3 cells, P=0.005). There was no significant difference in referential volumes of the granular cell layers between the two groups (control group: 0.919 ± 0.0515 mm3, DHA group: 0.920 ± 0.0547 mm3, P=0.993), the sizes of the granular cell layers were not affected.

### Example 1: Culture medium for neural stem cell

The final composition of a prepared medium for neural stem cells is described below.

### (1) Medium preparation example 1

10 µM DPA
0.1% fatty acid-free bovine serum albumin
DMEM/F12 (1:1)
N-2 supplement

### (2) Medium preparation example 2

10 µM DHA
0.1% fatty acid-free bovine serum albumin
DMEM/F12 (1:1)
N-2 supplement

## Claims

1. A neuronal differentiation promoting agent for neural stem cells, comprising as an active ingredient at least one member selected from the group consisting of ω-3 unsaturated fatty acids and ω-6 unsaturated fatty acids having 18 to 22 carbon atoms, and derivatives thereof.

2. A neuronal differentiation promoting agent for neural stem cells, comprising as an active ingredient at least one member selected from the group consisting of eicosapentaenoic acid (EPA), docosahexaenoic acid (DHA), docosapentaenoic acid (DPA), and derivatives thereof.

3. The neuronal differentiation promoting agent according to Claim 1 or 2, which is used for promoting differentiation of neural stem cells into neurocytes.

4. The neuronal differentiation promoting agent according to any one of Claims 1 to 3, which is used for regenerative medicine for nerves.

5. A method of promoting differentiation of undifferentiated neural cells, comprising a step of adding the neuronal differentiation promoting agent according to any one of Claims 1 to 3.

6. The method according to Claim 5, which promotes differentiation of neural stem cells into neurocytes.

7. A composition for regenerative medicine, comprising as a main component a culture obtained by adding the neuronal differentiation promoting agent according to any one of Claims 1 to 4 to neural stem cells.

8. A culture medium for undifferentiated neural cells, comprising the neuronal differentiation promoting agent according to any one of Claims 1 to 3.
